Europäisches Patentamt

European Patent Office

Office européen des brevets

(19)

(11) **EP 1 547 472 A1**

(12) **EUROPEAN PATENT APPLICATION**
published in accordance with Art. 158(3) EPC

(43) Date of publication:
**29.06.2005 Bulletin 2005/26**

(51) Int Cl.⁷: **A23L 1/076**, A61K 7/00,
A61K 7/48, A23K 1/16

(21) Application number: **03794234.9**

(22) Date of filing: **04.09.2003**

(86) International application number:
**PCT/JP2003/011311**

(87) International publication number:
**WO 2004/021803 (18.03.2004 Gazette 2004/12)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **06.09.2002 JP 2002262163**

(71) Applicant: **Hayashibara, Ken
Okayama-shi Okayama700-0921 (JP)**

(72) Inventors:
• **SHIBUYA, Takashi,
c/o K. K. Hayashibara Seibutsu
Okayama-shi, Okayama 700-0907 (JP)**

• **FUKUDA, Shigeharu,
c/o K. K. Hayashibara Seibutsu
Okayama-shi, Okayama 700-0907 (JP)**
• **MIYAKE, Toshio, c/o K. K. Hayashibara Seibutsu
Okayama-shi, Okayama 700-0907 (JP)**

(74) Representative: **Daniels, Jeffrey Nicholas et al
Page White & Farrer
54 Doughty Street
London WC1N 2LS (GB)**

(54) **REFINED ROYAL JELLY**

(57)     The object of the present invention is to provide an allergen-reduced royal jelly, having less fear of causing allergic symptoms, obtained by reducing allergens and keeping the useful pharmacological actions of royal jelly, a process for producing the same, and its uses. The present invention solves the above object by providing an allergen-reduced royal jelly where the amount of water-soluble proteins is reduced to less than 50% (w/w) to the amount of total proteins; a process for producing the allergen-reduced royal jelly comprising the steps of separating a royal jelly into a precipitate and a supernatant comprising water-soluble proteins by adding water and centrifuging, collecting low molecular substances by eliminating high molecular substances comprising water-soluble proteins from the resulting supernatant using ultra-filtration or gel filtration chromatography, and mixing the resulting low molecular substances with the aforesaid precipitate; a composition comprising the allergen-reduced royal jelly; and the uses of the composition for foods, feeds, baits, pet foods, and cosmetics.

EP 1 547 472 A1

**Description**

## TECHNICAL FIELD

The present invention relates to an allergen-reduced royal jelly (Hereinafter, simply abbreviated as "ARRJ"), particularly, ARRJ where the amount of water-soluble proteins is reduced to less than 50% (w/w) to that of total proteins, a process for producing the same, and its uses.

## BACKGROUND ART

Royal jelly is a milk-white humor, secreted from cephalic glands of worker honeybees and stored in a royal cell (a cell for queen bee) of honeycomb, and a jelly given as a feed to larva which grows to queen bee. Although its chemical composition is varied in some degree depending on its source and season, it has been reported that the composition is 65-75% (w/w) of water, 15-20% (w/w) of proteins, 10-15% (w/w) of carbohydrates, 1.7-6% (w/w) of fats, and 0.7-2% (w/w) of ashes. Further, royal jelly comprises organic acids represented by 10-hydroxy-2-decenoic acid, various vitamins and minerals. It has been reported that proteins of royal jelly includes water-soluble proteins and water-insoluble proteins, and the contents of the former and the later are about 75% and 25% to the total proteins, respectively (for the example, refer to Tetsuo Takenaka, "*Mitsubachi Kagaku* (Honeybee Science)", Vol. 3, No.2, pp. 69-74, 1982).

Royal jelly has long been utilized widely as a healthy food for human. Recently, many reports have revealed that royal jelly has useful pharmacological actions for human body such as an antibacterial action, immune-enhancing action, anti-tumor action, anti-inflammatory action, and life-prolonging action.

However, since royal jelly contains allergic substances, it may cause an allergic symptom when it is consumed. Although the allergic symptom varies depending on a person, in some cases, royal jelly causes a significant anaphylatic shock. To overcome such disadvantage of royal jelly, Japanese Patent Kokai No. 112,715/2002 discloses a method for reducing allergens by treating royal jelly with saccharide-degrading enzymes and protein-degrading enzymes. However, in the case of such royal jelly produced by the steps of precipitating allergic water-soluble proteins by isoelectric point precipitation and degrading the resulting precipitate by saccharide-degrading enzymes and protein degrading enzymes, the additional enzymes themselves would possibly be allergic substances. Therefore, the above royal jelly has a disadvantage of not being preferable as royal jelly for keeping health.

## DISCLOSURE OF INVENTION

The present invention was accomplished under these circumstances. The object of the present invention is to provide ARRJ having less fear of causing allergic symptom by reducing allergens and keeping the useful pharmacological actions of royal jelly, a process for producing the same, and the uses of a composition comprising ARRJ for foods, feeds, baits, pet foods, and cosmetics.

To solve the above object, the present inventors have extensively studied on a high quality ARRJ having less fear of causing allergic symptom by reducing allergens and keeping the useful pharmacological actions of royal jelly and a process for producing AARJ. As a result, the present inventors found that the most part of allergic substances, comprised in a fresh royal jelly, are water-soluble and contained in supernatant by the steps of admixing water with non-treated royal jelly (hereinafter, "non-treated royal jelly" may be simply abbreviated as "royal jelly" or "fresh royal jelly" in this specification. Further, it may be abbreviated as "RJ" in Tables.) as a material, separating the mixture into a supernatant and a precipitate by centrifugation, and measuring the allergic activity of those using a fresh royal jelly as a control. The present inventors also found as original knowledge that ARRJ, where the amount of water-soluble proteins is reduced to less than 50% (w/w) (hereinafter, "% (w/w)" is simply abbreviated as "%" in this specification) to the amount of total proteins and the amount of allergens is reduced, which keeps the useful pharmacological actions of fresh royal jelly, can be produced by a method constructed by combining the processes of admixing water with a fresh royal jelly and separating the mixture into a supernatant and a precipitate by centrifugation (hereinafter, the process is abbreviated as "process of water-fractionation" in this specification); collecting low molecular substances by eliminating high molecular substances comprising water-soluble proteins from the resulting supernatant using ultra-filtration or gel-filtration chromatography (hereinafter, the process is abbreviated as "process of high molecular substances elimination" in this specification); and mixing the resulting low molecular substances with the aforesaid precipitate (hereinafter, the process is abbreviated as "process of mixing" in this specification).

Specifically, the present invention solves the above object by providing ARRJ, where the amount of water-soluble proteins is reduced to less than 50% to the amount of total proteins, the process for producing the same, a composition comprising ARRJ, and the uses of the composition for foods, feeds, baits, pet foods, and cosmetics.

## BEST MODE FOR CARRYING OUT THE INVENTION

The present invention relates to ARRJ where the amount of water-soluble proteins is reduced to less than 50% to the amount of total proteins. A fresh royal jelly usable as a material for ARRJ of the present invention is not restricted to species of bee secreting it, sources, and its forms (raw or frozen form) supplied. A royal jelly usable in the present invention is those secreted by *Apis mellifera, Apis cerana, Apis dorsata, Apis florae*, etc. A royal jelly usable in the present invention is obtained from a source in Japan, South America, North America, Australia, China, Europe, etc. Although all those royal jellies can be used as materials for ARRJ of the present invention, it is preferable to use royal jelly as fresh as possible or that preserved under a relatively low temperature. In ARRJ of the present invention, since the amount of water-soluble proteins is reduced to less than 50%, preferably, less than 40%, more preferably, less than 20%, most preferably, less than 10% to the amount of total proteins, the allergic activity is reduced. In the present invention, the amount of proteins is determined by the method of Lowry (O. H. Lowry *et al., Journal Biological Chemistry,* Vol. 193, 265, 1951), a method for protein determination used generally in the art, using bovine serum albumin as a standard protein. While, the allergic activity of royal jelly can be determined by the method described in the following Experiments.

ARRJ of the present invention well keeps 10-hydroxy-2-decenoic acid having the useful pharmacological actions of royal jelly, while high molecular substances, i.e., proteins, which are a cause of allergic activity, are reduced. The weight ratio of total proteins to 10-hydroxy-2-decenoic acid, on a free fatty acid basis, can be used for the physicochemical index of ARRJ; and that for ARRJ of the present invention is less than six. Since the ratio of a fresh royal jelly is usually in the range of 7 to 15, the value can be clearly discriminated from that of ARRJ of the present invention. The amount of 10-hydroxy-2-decenoic acid can be determined by the method described in the following Experiments.

Also, ARRJ of the present invention can be identified by the steps of applying the protein of ARRJ to isoelectrofocusing in the range of pH 3-10 in the presence of 8M urea and measuring the amount ratio of proteins showing the isoelectric point of less than 5 and that of 5 or higher. Since the value of ARRJ of the present invention is one or higher, ARRJ can be discriminated from a fresh royal jelly showing the value of less than one.

Various methods for purifying royal jelly can be arbitrarily used as far as they can reduce allergic activity without substantially losing the useful pharmacological actions of royal jelly. Concretely, the method, where the amount of water-soluble proteins can be reduced to less than 50%, preferably, less than 40%, more preferably, less than 20%, most preferably, less than 10% to the amount of total proteins, is desirable. As concrete purification methods, one or more methods generally used in the art for purifying substances comprising proteins such as dilution with water, centrifugation, filtration with membrane, filtration, concentration, separatory precipitation, salting out, dialysis, ion-exchange chromatography, gel filtration chromatography, adsorption chromatography, chromatofocusing, hydrophobic chromatography, reverse-phase chromatography, affinity chromatography, gel electrophoresis, and isoelectrofocusing can be arbitrarily used.

The process for producing ARRJ of the present invention is constructed by the combination of processes of water-fractionation, high molecular substance-elimination, and mixing. In the above purification methods, dilution with water, and centrifugation are used in a process of water-fractionation. Although membrane fractionation and gel filtration chromatography can be used in a process of high molecular substance-elimination, membrane fractionation is more preferable because of its convenience.

In the present invention, water-soluble proteins are comprised in a supernatant obtained by the process of water-fractionation comprises the steps of admixing water to fresh royal jelly and centrifuging. Therefore, they can be separated from a water-insoluble precipitate. The amount of total proteins as referred to as in the present invention means sum of the amount of proteins in the supernatant (the amount of water-soluble proteins) and that in the precipitate as calculated by formula 1. The content (%) of water-soluble proteins in total proteins as referred to as in the present invention means the value calculated by formula 2.

Formula 1

Total proteins

= Proteins in the supernatant (Water-soluble proteins)

+ Proteins in the precipitate

Formula 2

The content (%) of water-soluble proteins in total proteins

= (Water-soluble proteins/Total proteins) x 100

The following explains each process for purifying royal jelly of the present invention.

Process of water-fractionation

As aforementioned, the present inventors found that the allergic activity in royal jelly mainly originated from high molecular substances which present in a supernatant obtained by water-fractionation of royal jelly. The process is to separate royal jelly into a supernatant and a precipitate by water-fractionation. Water to be admixed with royal jelly is not restricted, and optionally, any water such as ultra-pure water, deionized water, distilled water, magnetized water, mineral water, running water, and deep-sea water can be used. Optionally, buffers can be used to keep pH within a prescribed range of aqueous solution in which royal jelly is suspended or dissolved. Since the pH of aqueous solution in which royal jelly is suspended or dissolved is usually in the range of 3.5-4.5, it is not necessary to control pH of the solution. However, in the case of being the pH lower than 3.5, the solution shows relatively strong sourness and is inconvenient for processing as foods. Also, in the case of being the pH higher than 4.5, the solution is not preferable for water-fractionation because inherently water-soluble proteins may form precipitate by isoelectric point precipitation. It is desirable to keep the pH of aqueous solution in which royal jelly is suspended or dissolved in the range of 3.5-4.5. To stabilize effective components comprised in royal jelly, the use of sterile water with a relatively low temperature is desirable. Although the amount of water to be admixed with royal jelly is varied depending on repeating times of water-fractionation, usually, one to 1000-folds, preferably, one to 100-folds of water to the weight of a fresh royal jelly used. In the case of using only a little amount of water, the dissolving of water-soluble proteins will be insufficient and the separation of a fresh royal jelly into a supernatant and a precipitate will also be insufficient. In the case of using too large amount of water, it causes the deterioration of effective components and is not preferable from the view point of working efficiency because it requires a longer production time. Water-fractionation can be done by using a relatively large amount of water and centrifuging at a time or by using a relatively small amount of water and centrifuging for several times. For the purpose of reducing the content of water-soluble proteins in total proteins to less than 50%, it is desirable to apply water-fractionation at least once, preferably, twice or more. Methods for centrifugation are not restricted as far as they can separate the suspension into a supernatant and a precipitate well, and both batch- and continuous-modes can be advantageously used. Although the condition of centrifuging is varied depending on centrifugal force and running time, it is desirable to centrifuge, usually, 3,000 x g for 10 minutes or longer, preferably, 5,000 x g for 10 minutes or longer, more preferably, 10,000 x g for 10 minutes or longer.

Process of eliminating high molecular substances

The above supernatant, showing an allergic activity, obtained by the water-fractionation comprises water-soluble proteins as high molecular substances. The supernatant also comprises low molecular substances such as organic acids including 10-hydroxy-2-decenoic acid, amino acids, saccharides, and vitamins. The process is carried out for separating substances present in the supernatant into high molecular substances and low molecular substances, eliminating high molecular substances comprising water-soluble proteins, and collecting useful low molecular substances. Water-soluble proteins as high molecular substance can be effectively eliminated by membrane-separation using an ultrafiltration membrane which has a pore size of not allowing to pass water-soluble proteins. For such an ultrafiltration membrane, having a molecular weight cut off of 1, 000-10 , 000 daltons, is preferable. In the case of using a membrane having a molecular weight cut off of less than 1,000 daltons, there is a fear of being also eliminated low molecular substances. In the case of using a membrane having a molecular weight cut off of higher than 10, 000 daltons, there is a fear of infecting a part of water-soluble proteins as high molecular substances. Since it is possible to separate into low molecular substances and high molecular substances by gel filtration chromatography, such a method can also be applied to the process of eliminating high molecular substances of the present invention. However, with regard to working efficiency and industrial applicability, it is desirable to use ultrafiltration membranes.

Process of mixing

The process is carried out for producing ARRJ of the present invention by mixing useful low molecular substances, collected by the process of eliminating high molecular substances, with the aforesaid precipitate. Low molecular substances, obtained by ultrafiltration or gel filtration, can be directly mixed with the aforesaid precipitate. In many cases, low molecular substances, obtained by ultrafiltration or gel filtration, are considerably diluted in comparison with those comprised in the material. In such case, the low molecular substances can be optionally concentrated and mixed with the precipitate. Optionally, after mixing the low molecular substances with the aforesaid precipitate, the mixture can be concentrated. Methods for such concentration can be arbitrarily selected from heating, concentrating under a reduced pressure, and a technique using a reverse osmosis membrane. Among these methods, concentration under a reduced pressure and that using a reverse osmosis membrane are preferable because they have less fear of heat-denaturing of effective components comprised in royal jelly. In the case of concentrating with a reverse osmosis membrane, the use of the membrane which is able to collect water-soluble low molecular substances comprised in royal jelly to the concentrate fraction, is preferable. It is desirable to used a reverse osmosis membrane having a sodium chloride cut off rate of, usually, 75% or higher, preferably, 90% or higher, more preferably, 95% or higher. AARJ of the present invention, having desirable concentration, can be obtained by adjusting the concentration using the procedure.

In AARJ of the present invention obtained by the above procedure, the content of water-soluble proteins in total proteins is reduced to less than 50%. Accompanying with the reduction, the amount of allergic substances is reduced but the useful pharmacological actions such as an anti-inflammatory action and life-prolonging action, those are inherently exhibited in royal jelly, are not lost. Therefore, AARJ of the present invention can be advantageously used as a healthy food for the purpose of keeping and promoting health.

While AARJ of the present invention can be used intact as described above, it can be advantageously used in a form of composition produced by incorporating it into other ingredients. The present invention also provides such compositions. A composition of the present invention usually comprises one or more ingredients, which are allowed to use for oral- or transdermal-applications or external use for skin, to mammals including human. Therefore, such composition can be advantageously used in the fields of foods, beverages, feeds, baits, pet foods, cosmetics and the like. An ingredient useable for oral- or transdermal- applications or external use for skin, to mammals including human, includes substances generally used in each field of the composition of the present invention such as water, alcohols, starchy substances, amino acids, fibers, saccharides, fats, fatty acids, vitamins, minerals, flavors, colorings, sweeteners, seasonings, spices, preservatives, emulsifiers, detergents, and the like. The form of the composition comprising above ingredients is not restricted. Therefore, the composition of the present invention can be provided in a desirable form such as a powder, granule, tablet, paste, emulsion and liquid.

A composition comprising ARRJ of the present invention can be produced by the steps of mixing above ingredients with ARRJ based on respective content according to the object, i.e. , based on an arbitrary composition selected according to the objective mammal and the method of administration; applying an arbitrary treatment such as dilution, concentration, drying, filtration, centrifugation, and the like; and optionally shaping into a desirable form. The order of incorporating each ingredient and the timing for applying various treatments are not restricted as far as they do not causing the deterioration of ARRJ. For example, it is preferable that ARRJ just after prepared or preserved under a low temperature after preparation is incorporated into the ingredients and then optionally applied to various treatments. For preventing the deterioration of ARRJ in the production process, it is preferable that the above all processes are carried out under ambient temperature, preferably, under the condition of 30°C or lower.

A composition comprising ARRJ of the present invention can be processed into a solid form by incorporating it into an anhydrous saccharide and drying. Further, ARRJ can be processed into a form of powder, granule, tablet, and the like. As anhydrous saccharide, anhydrous $\alpha,\alpha$-trehalose, anhydrous maltose, anhydrous cyclic tetrasaccharide, and the like can be used. Anhydrous $\alpha,\alpha$-trehalose can be produced easily from "TREHA®", commercially available crystalline trehalose dihydrate commercialized by Hayashibara Shoji Inc. , Okayama, Japan, by the method disclosed in Japanese Patent No. 3,168,550, and used for the above object. A commercially available anhydrous crystalline maltose, "FINETOSE®", commercialized by Hayashibara Shoji Inc., Okayama, Japan, can be used as anhydrous maltose. Further, anhydrous cyclic tetrasaccharide disclosed in WO 02/057,011 can be advantageously used.

A composition, produced by incorporating ARRJ into an anhydrous saccharide of the present invention, can be obtained by the steps of mixing ARRJ and an anhydrous saccharide, optionally, further admixing other ingredients with the mixture, drying the resulting mixture by allowing to absorb water to anhydrous saccharide, and optionally further subjecting to general drying process such as drying under reduced pressure, drying *in vacuo*, and heating. More concretely, ARRJ is admixed with, usually, four-folds or more amounts by weight, preferably, eight-folds or more amounts by weight to the amount of ARRJ of crystalline or amorphous anhydrous saccharide, and optionally the mixture is further admixed with other ingredients. Successively, the resulting mixture is dehydrated and dried by keeping under ambient temperature or lower, preferably, 30°C or lower for, usually, four hours or longer, preferably, eight hours or longer. Optionally, ARRJ in a solid form, thus prepared, is further dried. ARRJ in a solid form can be advantageously processed to a desirable form such as a powder, granule, and tablet by using a pulverizer, granulator, and tableting machine. Optionally, the resulting powder or granule can be advantageously used after filling into a capsule.

Optionally, a commercially available saccharide-transferred vitamin C (alias L-ascorbic acid 2-glucoside) can be admixed with a composition of the present invention described above. It has been well known that L-ascorbic acid has the function of enhancing the production of collagen in living bodies. However, L-ascorbic acid has disadvantages of being unstable and being easily decomposed by oxidation. While, the saccharide-transferred vitamin C is a chemically stable substance and hydrolyzed in living bodies to release L-ascorbic acid.

Effective components comprised in ARRJ can be further stabilized by incorporating antioxidant substances into a composition of the present invention. Therefore, antioxidant substances can be advantageously used according to the object or area of the application, for example, in the case of transporting the composition by ship and the like without controlling the temperature or using the composition in an area with high temperature. Antioxidant substances usable in the present invention are not restricted specifically. However, in the case of using the composition as edible one for mammal including human, it is preferable of selecting edible antioxidant substances in the field of foods. Concrete edible antioxidant substance, generally used in the field of foods, includes flavonoids, polyphenols, vitamin E and vitamin C. Flavonoids include rutin, hesperidin, naringin, quercetin, and those derivatives, produced by transferring glucose or its polymer (oligosaccharide), such as saccharide-transferred rutin, saccharide-transferred hesperidin, saccharide-transferred naringin, and saccharide-transferred quercetin. Catechin and gallic acid can be used as polyphenols. Further, extracts of plants such as *Sophora japonic L.* extract, rosemary extract, and eucalyptus extract can be used in the present invention as antioxidant substances. The content of these antioxidant substances in the composition is not restricted. However, in the case of using the composition as a food, it is preferable to use it according to the amount generally used in the field of foods or less than that.

A desirable form of the composition of the present invention as foods includes, for example, frozen dessert such as ice cream, ice candy, and sherbet; syrup such as "*korimitsu*" (a sugar syrup for shaved ice), spreads and pastes such as butter cream, custard cream, flour paste, peanut paste, and fruit paste; confectionery such as chocolate, jelly, candy, gummy jelly, caramel, chewing gum, custard pudding, cream puff, sponge cake, and the like; processed fruit or vegetable such as jam, marmalade, "*syrup-zuke*" (fruit pickles), and "*toka*" (conserves), and the like; Japanese confectionery such as "*manju*" (a bun stuffed with *azuki*-bean paste), "*uiro*" (a sweet rice jelly), "*an*" (*azuki*-bean jam), "*yokan*" (a sweet bean jelly), "*mizuyohkan*" (a soft sweet bean jelly), pao de Castella, "*amedama*" (a Japanese toffee), and the like; and seasoning such as soy-sauce, powderized soy-sauce, "*miso*" (soy-bean paste), powdered "*miso*", mayonnaise, dressing, vinegar, "*sanbai-zu*" (a sauce of sugar, soy sauce and vinegar), table sugar, coffee sugar, and the like. Desirable form as beverage of the composition of the present invention includes, for example, alcoholic beverage such as synthetic *sake*, fermented liquor, fruit liquor, and *sake;* and soft drink such as juice, beverage containing minerals, carbonated beverage, sour milk beverage, beverage containing a lactic acid bacterium, isotonic drink, nutritional drink, green-tea, tea, oolong tea, coffee, cocoa, and the like.

ARRJ of the present invention can be also advantageously used intact or in the form of composition comprising other ingredients for a feed, bait, pet food for animals such as domestic animals, poultry, pets, honey bees, silk warms, insects, and fishes. As other ingredients which can be incorporated into compositions having such forms, one or more ingredients, usually used in the respective fields of feeds, baits, and pet foods, for example, grains, starchy substances, starch hydrol, saccharides, fats, nuts, beans, fish and shellfish, meats, eggs, milks, extract of plant protein, fruits, mushrooms, algae, vitamins, minerals, amino acids, yeast, grasses, bagasse, corncob, rice straw, hays, oil cakes, bran, soybean-bran, and various fermented bran can be used. The composition can be advantageously used in solid form such as powder, granule, tablet, paste, gummy, and the like, and liquid form such as emulsion and drink.

A desirable form of the composition of the present invention as cosmetics includes basic skin care cosmetics, cosmetics for washing, cosmetics for bath, hair care cosmetics, suntan and sunscreen cosmetics, makeup cosmetics, hair growth stimulants, and hair growth tonics.

To produce a composition of the present invention in the form of above, ARRJ of the present invention can be incorporated into the composition at arbitrary timing in the process of producing the objective product according to the conventional method. The timing of incorporating ARRJ is not specifically restricted. However, in the case of producing the objective product through a heating process, the decreasing of the useful pharmacological action of ARRJ in the production process can be prevented by incorporating ARRJ into the product after cooling that to ambient temperature, preferably, 30°C or lower after the heating process. A composition of the present invention described above comprises ARRJ of the present invention, usually, 0.001-20%, preferably, 0.01-10%, on a weight of product basis.

In the composition of the present invention described above, since the allergic activity is reduced and various useful pharmacological actions are stabilized, the useful pharmacological actions are effectively expressed in living bodies and the composition can be used for increasing resistance of living bodies, improving bad condition quickly, and keeping good health. Accordingly, the composition of the present invention is very useful as foods, beverages, feeds, baits, pet foods, cosmetics, and the like.

The following experiments explain the present invention in detail:

Experiment 1

Water-fractionation of Brazilian and Chinese royal jellies and test for allergic activity

Experiment 1-1

Water-fractionation of Brazilian and Chinese royal jellies

Brazilian fresh royal jelly (moisture content of 62.5%) and Chinese fresh royal jelly (moisture content of 65.7%), those are frozen and preserved, were thawed and weighed 12 grams each. Forty-eight grams, four-folds amount by weight to the weighed royal jelly, of sterile deionized water was admixed with above royal jelly and stirred to homogeneity. After confirming the uniform dispersion, the suspension was centrifuged at 5,000 x g for 10 minutes, and the resulting supernatant and precipitate were collected, respectively. As a control, 12 grams of a fresh royal jelly and 48 grams of water were mixed, and then stirred and dispersed similarly as above.

Experiment 1-2

Test for allergic activity

On the evaluation of allergic activity comprised in a supernatant and a precipitate prepared from Brazilian and Chinese royal jellies in Experiment 1-1, the amount of all samples was used by adjusting the amount corresponding to fresh royal jelly. The amount corresponding to fresh royal jelly as referred to as in the present invention means the amount of sample converted to the weight of material fresh royal jelly, and the amount of sample originated from one gram of inherent fresh royal jelly is expressed to one gram corresponding to fresh royal jelly in each case of supernatant or precipitate. Test for allergic activity was carried out using an antigenicity test for mice according to the method of Hagita *et al*. (Tadaatsu Hagita and Hiroshi Mizushima, "*Igaku no Ayumi* (Proceeding of Medical Science)", Vol. 100, pp. 814, 1977). Specifically, 1.5 mg corresponding to fresh royal jelly of each sample and 2.5 mg of aluminum hydroxide gel (Alum) were administrated to peritoneal cavity of five female BALB/c mice (nine-weeks-old, commercialized by Charles River Japan Inc. , Kanagawa, Japan) per group with three times every other week. One week later from the third administration, blood was collected from the caudal vein of each mouse and then serum was collected using a centrifuging tube and subjected to PCA method. Specifically, 0.1 ml each of mouse serum diluted to 100- or 200-folds with saline was used for intradermal injection to the dehaired-back of SD mouse. After 24 hours from the injection, 7.5 mg corresponding to fresh royal jelly of preparation identical with that administrated to mouse and one ml of 0.5% Evan's Blue solution were used for intravenous injection, and the size of blue spot observed at the position of injecting serum was measured. A mouse, which shows the spot having an average diameter of 5 mm or higher, was judged as "positive". The strength of allergic activity was estimated to be "+++++" where all five mice in a test group are "positive" and "-" where all five mice are "negative". The results for supernatant, precipitate, and control of Brazilian royal jelly and those of Chinese royal jelly were in Table 1 and 2, respectively.

Table 1

| | Allergic activity | |
|---|---|---|
| Serum-dilution factor | 100-folds | 200-folds |
| Control (Fresh royal jelly) | +++ | - |
| Supernatant | +++++ | ++ |
| Precipitate | + | - |

Table 2

| | Allergic activity | |
|---|---|---|
| Serum-dilution factor | 100-folds | 200-folds |
| Control (Fresh royal jelly) | +++++ | +++ |
| Supernatant | +++++ | +++++ |
| Precipitate | ++++ | + |

As is evident from the results in Table 1 and 2, a relatively strong allergic activity was detected in a supernatant in both cases of Brazilian royal jelly and Chinese royal jelly. On the contrary, allergic activities in precipitate were relatively weak. It was supposed that water-soluble proteins were major allergic substances. Further, from the comparison of the results obtained by serum-dilution factor of 100- and 200-folds, it was revealed that the allergic activity of Chinese royal jelly is stronger than that of Brazilian royal jelly.

Experiment 2

Effects of repeating time of water-fractionation on the content of water-soluble proteins to the total proteins recovered in precipitate

by water-fractionation and the reduction of allergic activity

Experiment 2-1

Effects of repeating time of water-fractionation on the content of water-soluble proteins to the total proteins recovered in precipitate by water-fractionation

To examine the effects of repeating time of water-fractionation on the content of water-soluble proteins to the total proteins recovered in precipitate, 12 grams of Brazilian royal jelly (moisture content of 62.5%) was used as material. Similarly to the case of Experiment 1-1, four-folds amount by weight (48 grams) of water was admixed with the material and the resulting mixture was separated into a supernatant and a precipitate by centrifuging at 5,000 x g for 10 minutes. To the resulting precipitate, same water-fractionation procedure was repeated two-times to prepare precipitate. In order to calculate the content of water-soluble proteins comprised in each precipitate obtained by one-, two-, or three-times water-fractionation, four-folds amount by weight of water was admixed with each precipitate similarly to the above water-fractionation. After centrifuging, the amount of proteins in the supernatant and that in precipitate were determined by the method of Lowry *et al*. using bovine serum albumin as a standard protein. The amount of proteins in the supernatant was defined as the amount of water-soluble proteins. As a control, a fresh royal jelly suspension prepared as in the case of Experiment 1-1 was centrifuged and the amount of proteins was determined in a same manner with above precipitate. Assay data of total proteins and water-soluble proteins comprised in precipitate recovered from each water-fractionation times and the content of water-soluble proteins in total proteins calculated from those data are in Table 3.

Table 3

| Water-fractionation (Times) | Proteins in supernatant (mg) | Proteins in precipitate (mg) | Total proteins (mg) | Content of WSP* (%) |
|---|---|---|---|---|
| Control (Fresh RJ**) | 1092.0 | 567.0 | 1569.0 | 65.8 |
| 1 | 272.0 | 424.8 | 696.8 | 39.0 |
| 2 | 25.9 | 412.8 | 438.7 | 5.9 |
| 3 | 16.5 | 387.6 | 404.1 | 4.1 |

*, Water-soluble proteins
**, Royal jelly

As is evident from the results in Table 3, the content of water-soluble proteins to total proteins in fresh royal jelly was 65.8%. While, the values of the precipitates obtained by one- and two-times water-fractionation were reduced to about 40% and about 6%, respectively.

Experiment 2-2

The relationship between the precipitate obtained by water-fractionation and its allergic activity

Allergic activity of precipitates, obtained by the water-fractionation of one-, two-, and three times in Experiment 2-1, was determined by the same method of Experiment 1-2 except for altering the serum-dilution factor to 50- and 100-folds. A suspension of fresh royal jelly prepared by the same method of Experiment 1-1 was used as control. The results are in Table 4.

Table 4

| | Allergic activity | |
| --- | --- | --- |
| Water-fractionation (Times) | Serum-dilution factor | |
| | 50-folds | 100-folds |
| Control (RJ*) | +++++ | +++ |
| 1 | ++ | + |
| 2 | + | - |
| 3 | - | - |

*, Royal jelly

From above results, it was revealed that the content of water-soluble proteins in total proteins of precipitate can be reduced to less than about 40% and the allergic activity can be remarkably reduced by applying the water-fractionation once.

Experiment 3

Preparation of various preparations from royal jelly, and ARRJ

Sixty grams of Chinese fresh royal jelly as material and 240 grams of deionized water at 4° C were mixed and stirred to give homogenous dispersion. Successively, the suspension was centrifuged (5,000 x g for 10 minutes) and separated into a supernatant and a precipitate. The resulting precipitate was mixed with 240 grams of deionized water at 4° C again and treated the suspension as above to obtain a precipitate. A supernatant of water-fractionation was prepared by mixing a supernatant obtained by the first water-fractionation and that by the second and subjected to ultrafiltration (UF) using "AIP0013" , a pencil type ultrafiltration membrane having a molecular weight cut off of 6 , 000 daltons, commercialized by Asahi Kasei Corporation, Tokyo, Japan. The supernatant was separated into a UF-filtrate and a UF-concentrate. Finally, ARRJ was prepared by mixing the resulting UF-filtrate and the aforesaid precipitate. The amounts of 10-hydroxy-2-decenoic acid, proteins and saccharides comprised in samples of each preparation step were determined. 10-Hydroxy-2-decenoic acid is a specific component of royal jelly as an index of effective component and reported to be involving saccharide metabolism and prevention of aging. The amount of proteins was determined by the method of Lowry *et al*. using bovine serum albumin as a standard protein. The amount of saccharides was determined by anthrone-sulfuric acid method using glucose as a standard saccharide. The amount of 10-hydroxy-2-decenoic acid was determined by HPLC method using the following conditions.

HPLC conditions for the determination of 10-hydroxy-2-decenoic acid

Column : YMC-Pack AQ-303-ODS ($\phi$ 4.6 mm x 250 mm, YMC Co., Ltd.)
Column temperature : 40°C
Detection : UV 210 nm
Mobile phase : 50% methanol (pH was adjusted to 2.2 by phosphoric acid)
Flow rate: 0.6 ml/minute
Sample volume : 20 $\mu$l
The amount of 10-hydroxy-2-decenoic acid was determined by the standard curve prepared using standard 10-hydroxy-2-decenoic acid solutions of 20, 50, and 100 $\mu$g/ml.
Recovery (%) of each component at each preparation step was calculated using their amounts comprised in material fresh royal jelly as 100%, respectively. The results are in Table 5.

EP 1 547 472 A1

Table 5

| Preparation No. | Preparation | Weight of solution (g) | Proteins | | 10-HDA**** | | Saccharides | |
|---|---|---|---|---|---|---|---|---|
| | | | (mg) | Yield (%) | (mg) | Yield (%) | (mg) | Yield (%) |
| 1 | Fresh RJ* | 60 | 8280 | 100 | 1145 | 100 | 8580 | 100 |
| 2 | Precipitate | 275 | 2305 | 28 | 275 | 24 | 165 | 2 |
| 3 | Supernatant | 500 | 6605 | 80 | 800 | 70 | 8620 | 100 |
| 4 | UF**-filtrate | 600 | 195 | 2 | 660 | 58 | 7820 | 91 |
| 5 | UF-concentrate | 250 | 5830 | 70 | 5 | 1 | 915 | 11 |
| 6 | ARRJ*** | 875 | 2500 | 30 | 935 | 82 | 7985 | 93 |

\*, Royal jelly

\*\*, Ultra-filtration

\*\*\*, Allergen-reduced royal jelly of the present invention

\*\*\*\*, 10-hydroxy-2-decenoic acid

As is evident from Table 5, water-soluble proteins, which account for major part, 6,605 grams, of proteins comprised in royal jelly, were moved to a supernatant by water-fractionation, and it was revealed that 5,830 grams of water-soluble proteins, about 90% of total water-soluble proteins, can be eliminated as a UF-concentrate. It is also revealed that saccharides and useful low molecular substances represented by 10-hyfroxy-2-decenoic acid, comprised in material fresh royal jelly were moved to supernatant together with water-soluble proteins having a relatively strong allergic activity, and can be recovered in ARRJ of the present invention which is produced by mixing the precipitate and the UF-filtrate with the recovery of about 90% or higher and 80% or higher by the steps of applying the supernatant to UF-filtration, collecting the resulting filtrate, and admixing the filtrate with the precipitate. The weight ratio of total proteins to that of 10-hydroxy-2-decenoic acid, on a free-fatty acid basis, was about 2.7. The value was clearly different from that of material fresh royal jelly of about 7.2.

Experiment 4

Allergic activity and useful pharmacological action of various preparations from royal jelly and ARRJ

Allergic activity and activity for suppressing the production of tumor necrosis factor-$\alpha$ (TNF-$\alpha$), an index of anti-inflammatory action, of various preparations obtained from royal jelly in Experiment 3 and ARRJ were investigated.

Experiment 4-1

Determination of activity for suppressing the production of tumor necrosis factor-$\alpha$ (TNF-$\alpha$)

1.5 milliliters of 4% thioglycolate medium was injected into the abdominal cavity of BALB/c mouse. After three days, peritoneal macrophages induced in BALB/c mouse were collected from the abdominal cavity. Successively, each preparation obtained in Experiment 3-1 was added to a medium with adjusting the amount to that corresponding to material fresh royal jelly, and lipopolysaccharide (LPS), interferon-$\gamma$ (IFN-$\gamma$), and BALB/c mouse peritoneal macrophage were admixed with the medium to give final concentrations of 1 $\mu$g/ml, 10 IU/ml, and 5 x 10$^5$ cells/well, respectively, and the mixture was put in a well. After cultivating for 48 hours, the resulting culture supernatant was collected and the amount of TNF-$\alpha$ produced was measured by solid-phase enzyme-linked immuno solvent assay (ELISA). As a control 1, the measurement was carried out by the same procedure using phosphate-buffer saline instead of various preparations from royal jelly. As a control 2, the measurement was carried out with the same manner using a fresh royal jelly with the same concentration. The relative activity for suppressing the production of TNF-$\alpha$ was represented by percentage using the difference of TNF-$\alpha$ activities of control 1 and 2 as 100. Allergic activities of each preparation from royal jelly and ARRJ were evaluated according to the method in Experiment 1-2. The results are in Table 6.

Table 6

| Preparation No. | Preparation Preparation | Allergic activity (serum dilution factor : 200-folds) | Relative activity for suppressing the production of TNF-$\alpha$ (%) |
|---|---|---|---|
| 1 | Fresh RJ* | +++ | 100 |
| 2 | Precipitate | + | 0 |
| 3 | Supernatant | +++++ | 100 |
| 4 | UF**-filtrate | - | 90 |
| 5 | UF-concentrate | +++++ | 10 |
| 6 | ARRJ*** | + | 90 |

*, Royal jelly

**, Ultrafiltration

***, Allergen-reduced royal jelly of the present invention

As is evident from the results in Table 6, substances showing an allergic activity were moved to UF-concentrate by water-fractionation and membrane separation. The allergic activity of ARRJ which is prepared by mixing the precipitate of water-fractionation and the UF-filtrate was reduced to 1/3 compared with that of material fresh royal jelly. Further, it was revealed that about 90 % of activity for suppressing the production of TNF-$\alpha$, which is an index of anti-inflammatory inherently exhibited in royal jelly, was recovered in ARRJ of the present invention from material fresh royal jelly. As described above, it was concluded that AARJ of the present invention is useful for keeping and increasing the health because the allergic activity inherently exhibited in royal jelly was reduced and whose useful pharmacological actions were kept well.

Experiment 5

Comparison of proteins comprised in ARRJ and fresh royal jelly by gel isoelectrofocusing

To compare proteins comprised in ARRJ prepared by the method of Experiment 3 with those comprised in material Chinese royal jelly, both samples were subjected to gel isoelectrofocusing. Forty micrograms each of ARRJ and fresh royal jelly was put into a test tube and dried *in vacuo*. The resultant was dissolved in 100 μl of a solution for isoelectrofocusing comprising 8 M urea, 4% (w/v) CHAPS, and 0.5% (w/v) "Pharmalyte®", a carrier ampholyte commercialized by Amersham Biosciences K. K., Tokyo, Japan, and then admixed with 150 μl of a solution comprising 8 M urea, 4% (w/v) CHAPS, 50 mM dithiothreitol, and 1% IPG buffer commercialized by Amersham Biosciences K. K. , Tokyo, Japan, to prepare a sample for electrophoresis. Successively, the sample was put in an exclusive container and "IMMOBILINE DryStrip, pH 3-10, 13 cm", a precast gel for isoelectrofocusing commercialized by Amersham Biosciences K. K., Tokyo, Japan, was soaked in the sample solution. The gel was swelled by allowing to absorb the sample with keeping at room temperature for 16 hours. Isoelectrofocusing was carried out using the resulting gel with a condition of 100 V for two hours, 300 V for five hours, and 3,500 V for 12 hours. After the isoelectrofocusing, proteins separated in the gel were stained by soaking the gel in a solution comprising 0.025 % (w/v) Coomassie Brilliant Blue (CBB) R-250, 40% (v/v) methanol, and 7% (v/v) acetic acid. Successively, the stained gel was destained by stirring in a solution comprising 40% (v/v) methanol and 7% (v/v) acetic acid for 30 minutes and that comprising 5% (v/v) methanol and 7% (v/v) acetic acid for five hours at room temperature.

Proteins, comprised in ARRJ or fresh royal jelly were roughly separated into two protein groups with a borderline of isoelectric point 5. Therefore, the percentages (%) of proteins showing an isoelectric point of lower than 5 and that of 5 or higher were compared by the steps of analyzing the destained gel with "DUAL-WAVELENGTH CHROMATO SCANNER CS-930", a gel scanner commercialized by Shimadzu Corporation, Kyoto, Japan, and measuring the area ratio of absorption peak at 600 nm. The average results of four independent tests for ARRJ and fresh royal jelly are in Table 7, respectively.

Table 7

| Sample | Percentage of protein (%)* | |
|---|---|---|
| | pI** lower than 5 | pI 5 or higher |
| ARRJ (present invention) | 43.3 | 56.7 |
| Fresh royal jelly | 58.3 | 41.7 |

*, Average of four independent tests
**, Isoelectoric point

As is evident from the results shown in Table 7, in the usual fresh royal jelly, the percentage of proteins showing isoelectric point of 5 or higher was about 42% of total proteins. While, that of ARRJ of the present invention was about 57%. The results suggest that water-soluble proteins, having a strong allergic activity, which are reduced by water-fractionation, comprise relatively large amount of proteins having isoelectric point of lower than 5. The feature of ARRJ of the present invention is the fact that the ratio of proteins showing isoelectric point of 5 or higher to that of lower than 5 is one or higher at gel isoelectrofocusing, which is carried out in the presence of 8 M urea and in the range of pH 3-10, described in this specification.

Although the following examples concretely explain the present invention, the present invention is not restricted by them:

Example A-1

Preparation of ARRJ

Ten kilograms of a Brazilian fresh royal jelly was mixed with 40 kilograms of deionized water with a temperature of 4°C and stirred to gave a homogenous dispersion. Successively, the suspension was continuously centrifuged at 10,000 x g and at flow rate of 10 L/hour using a centrifugal separator "Model KT-2000G", commercialized by Kubota Manufacturing Corporation, Tokyo, Japan, to obtain a supernatant and a precipitate. The resulting supernatant was subjected to ultrafiltration using "AIP 2013", a UF-membrane having a molecular weight cut off of 6,000 daltons, commercialized by Asahi Kasei Corporation, Tokyo, Japan. The supernatant was separated into a UF-filtrate and a UF-concentrate using a small amount of deionized water as a dilution solvent. About 40 L of the resulting UF-filtrate was concentrated to give a weight of about 5 kilograms using "HOLLOSEP®", a reverse osmosis membrane commercialized by Toyobo Co., Ltd., Osaka, Japan, at a pressure of 50 kgf/cm$^2$. The resulting concentrate was admixed with the aforesaid precipitate obtained by the centrifugation and stirred to homogeneity to produce ARRJ. In ARRJ, the weight ratio of total proteins to 10-hydroxy-2-decenoic acid, on a free-fatty acid basis, was 3.7. By the analysis with gel isoelectrofocusing, the amount ratio of proteins showing an isoelectric point of 5 or higher to that of lower than 5 was about 1.3.

Example A-2

Preparation of ARRJ

Twenty kilograms of a Chinese fresh royal jelly was mixed with 80 kilograms of deionized water with a temperature of 4°C and stirred to gave a homogenous dispersion. Successively, the suspension was continuously centrifuged at 10,000 x g and at flow rate of 10 L/hour using a centrifugal separator "Model KT-2000G", commercialized by Kubota Manufacturing Corporation, Tokyo, Japan, to obtain a supernatant and a precipitate. The resulting precipitate was admixed with 80 kilograms of deionized water with a temperature at 4° C again and treated with same manner described above to obtain a precipitate. The first and the second supernatant obtained by above water-fractionation were mixed and the resulting solution was subjected to ultrafiltration using "AIP 2013", a UF-membrane having a molecular weight cut off of 6,000 daltons, commercialized by Asahi Kasei Corporation, Tokyo, Japan. The solution was separated into a UF-filtrate and a UF-concentrate using a small amount of deionized water as a dilution solvent. About 160 L of the resulting UF-filtrate was concentrated to give a weight of about 12 kilograms using "HOLLOSEP®", a reverse osmosis membrane commercialized by Toyobo Co., Ltd., Osaka, Japan, at a pressure of 50 kgf/cm$^2$. The resulting concentrate was admixed with the aforesaid precipitate obtained by the centrifugation and stirred to homogeneity to produce ARRJ. In ARRJ, the weight ratio of total proteins to 10-hydroxy-2-decenoic acid, on a free-fatty acid basis, was 4.2. By the analysis with gel isoelectrofocusing, the amount ratio of proteins showing an isoelectric point of 5 or higher to that of lower than 5 was about 1.2.

Example A-3

Preparation of ARRJ

Five parts by weight of a Chinese fresh royal jelly was mixed with 20 parts by weight of deionized water with a temperature of 4°C and stirred to gave a homogenous dispersion. Successively, the suspension was continuously centrifuged at 10,000 x g and at flow rate of 10 L/hour using a centrifugal separator "Model KT-2000G", commercialized by Kubota Manufacturing Corporation, Tokyo, Japan, to obtain a supernatant and a precipitate. The resulting precipitate was admixed with 20 parts by weight of deionized water with a temperature at 4°C again and treated with same manner described above to obtain a precipitate. The first and the second supernatant obtained by above water-fractionation were mixed and the resulting solution was subjected to gel filtration chromatography using "TOYOPEARL HW-40F" gel, a gel for gel filtration commercialized by Tosoh Corporation, Tokyo, Japan. The components were separated into a high molecular-fraction and a low molecular-fraction by eluting with deionized water. The resulting low molecular-fraction was concentrated to give eight parts by weight using "Model RE-10E-100", a reduced-pressure evaporator commercialized by Shibata Scientific Technology, Ltd., Tokyo, Japan, at 40°C. The resulting concentrate was admixed with the aforesaid precipitate obtained by the centrifugation and stirred to homogeneity to produce ARRJ. In ARRJ, the weight ratio of total proteins to 10-hydroxy-2-decenoic acid, on a free-fatty acid basis, was 4.9. By the analysis with gel isoelectrofocusing, the amount ratio of proteins showing an isoelectric point of 5 or higher to that of lower than 5 was about 1.25.

Referential example

Preparation of anhydrous α,α-trehalose

"TREHA®", a hydrous α,α-trehalose product commercialized by Hayashibara Shoji Inc. , Okayama, Japan, was dried under reduced pressure under the condition of at 90° C and barometric pressure at -300 to -350 mmHg for about seven hours using a rotary vacuum dryer with a jacket. After drying, anhydrous α,α-trehalose was obtained by the steps of reducing the temperature to ambient temperature, returning the barometric pressure to atmospheric condition, and collecting the product.

Example B-1

Healthy food

Five parts by weight of anhydrous $\alpha,\alpha$-trehalose obtained by the method of Referential example and one part by weight of ARRJ obtained by the method of Example A-1 were mixed to homogeneity for 15 minutes using "MODEL MDR-60", a multipurpose mixer commercialized by Dalton Corporation, Tokyo, Japan. After drying the mixture *in vacuo* at 40°C for overnight, the resultant was pulverized using "POWERMILL P-3", a mill commercialized by Dalton Corporation, Tokyo, Japan, with a screen size of 0.5 mm.

The powdery composition was shaped into tablets of about 200 mg/tablet using a tableting machine. The product is a composition which can be used easily and has a remarkable potency. Since the product shows a mild sweetness and moderate sourness, it is a useful healthy food for daily use.

Example B-2

Healthy food

A composition, comprising ARRJ of the present invention in a powdery form, was prepared by the steps of mixing the following ingredients with following proportion and processing the mixture according to the procedure in Example B-1. After the preparation, it was confirmed by the experiment according to Experiment 1 that the composition has a reduced allergic activity.

| | |
|---|---|
| Anhydrous $\alpha,\alpha$-trehalose obtained by the method of Referential example | 8.5 parts by weight |
| ARRJ obtained by the method of Example A-2 | 0.5 part by weight |
| "$\alpha$G-HESPERIDIN PS", a saccharide-transferred hesperidin commercialized by Hayashibara Shoji Inc., Okayama, Japan | 0.5 part by weight |
| "PULLULAN PF-20", a pullulan product commercialized by Hayashibara Shoji Inc., Okayama, Japan | 0.5 part by weight |

The composition comprising ARRJ was shaped into tablets of about 300 mg/tablet using a tableting machine. The product is a composition where the allergic activity is reduced and has a remarkable potency. Since the product shows a mild sweetness and moderate sourness, it is a useful healthy food for daily use.

Example B-3

Healthy food

A composition, comprising ARRJ of the present invention in a powdery form, was prepared by the steps of mixing the following ingredients with following proportion and processing the mixture according to the procedure in Example B-1. After the preparation, it was confirmed by the experiment according to Experiment 1 that the composition has a reduced allergic activity.

| | |
|---|---|
| Anhydrous $\alpha,\alpha$-trehalose obtained by the method of Referential example | 7.5 parts by weight |
| ARRJ obtained by the method of Example A-3 | 1.0 part by weight |
| Maltitol | 1.3 parts by weight |
| L-Tryptophan | 0.2 part by weight |

The product is a composition comprising ARRJ where the allergic activity is reduced and has a remarkable potency. Since the product shows a good taste with mild sweetness and moderate sourness, it is a useful healthy food for daily use.

Example B-4

Ice cream

Eighteen parts by weight of fresh cream (fat content; about 46% (w/w)), seven parts by weight of skim milk, 51 parts by weight of milk, 10 parts by weight of sucrose, four parts by weight of "NYU-KA OLIGO®", a powdery product comprising lactosucrose, two parts by weight of pullulan, and two parts by weight of gum arabic were mixed and dissolved. After sterilizing the mixture by keeping at 70°C for 30 minutes, the resultant was emulsified and dispersed using a homogenizer, then rapidly cooled to 3-4°C and admixed with four parts by weight of ARRJ obtained by the method of Example A-2. After aging the mixture for one night, ice cream was obtained by freezing in a freezer.
The product is an ice cream showing a moderate sweetness and fine flavor and being useful for keeping and increasing health.

Example B-5

"*Amazake*"

Ten parts by weight of polished rice was admixed with water and boiled according to the conventional method. Successively, the boiled rice was cooled to 55°C and admixed with 30 parts by weight of "*koji*" prepared by the conventional method and 0.1 part by weight of sodium chloride and then kept at 50 to 55°C for eight hours. After homogenizing the mixture using a mixer and cooling to about 25°C, two parts by weight of ARRJ obtained by the method of Example A-1 was admixed with the homogenate, stirred, and packed into a package to produce "*amazake*".
The product is a high-quality "*amazake*" having a satisfactory color and flavor. Further, it is a useful drink for keeping and increasing health.

Example B-6

Healthy beverage

A composition was produced by mixing 500 parts by weight of "FINETOSE®", an anhydrous crystalline maltose product, 100 parts by weight of ARRJ obtained by the method of Example A-1, 190 parts by weight of powderized egg yolk, 200 parts by weight of skim milk, two parts by weight of "AA2G®", a saccharide-transferred vitamin C commercialized by Hayashibara Biochemical Laboratories Inc, Okayama, Japan, 4.4 parts by weight of sodium chloride, 1. 85 parts by weight of potassium chloride, four parts by weight of magnesium sulfate, 0.01 part by weight of thiamin, 0.1 part by weight of L-ascorbic acid sodium salt, 0.6 parts by weight of vitamin E acetate, and 0.04 part by weight of nicotinamide. Twenty-five parts by weight of the composition was admixed with 150 parts by weight of purified water, dissolved to give homogenous dispersion and enclosed 150 grams each in a brown glass bottle.
Since the product has a reduced allergic activity and is supplemented with nutrients, it is advantageously used as a healthy beverage for the purpose of keeping health, promoting growth, preventing disease, promoting cure, promoting the recovery from distress after sports. The product can be advantageously used as a composition for oral intake or per-tube administration for animals such as domestic animals as well as human.

Example B-7

Pet food (gummy)

Thirty parts by weight of sucrose was admixed with eight parts by weight of water and dissolved by heating. After mixing the resultant with 50 parts by weight of starch syrup and boiled to give a Brix value of 85 to 90°, the mixture was cooled to 80° C or lower. Successively, a solution, prepared by mixing seven parts by weight of gelatin and ten parts by weight of water and dissolving by heating, was admixed with above saccharide solution. Successively, the mixture was further admixed with two parts by weight of beef extract, three parts by weight of 50% (w/w) citric acid aqueous solution, one part by weight of ARRJ obtained by the method of Example A-1, and suitable amount of flavor, and stirred to homogeneity. The composition thus obtained was poured into a starch mold and solidified by preserving for one night to produce a pet food in a gummy form.
Since the product comprises ARRJ where the allergic activity is reduced and is supplemented with nutrients, it ca be advantageously used as a pet food for the purpose of keeping health, promoting growth, preventing disease, and promoting cure of pets. The product can be advantageously used as a composition for oral intake for animals such as various pets and domestic animals as well as dogs and cats.

Example B-8

Skin cream for external use

The following ingredients were conventionally mixed with heating according to the following proportion.

| | |
|---|---|
| Polyoxyethlene-glycerin monostearate | 2.0 parts by weight |
| Self-emulsifying glycerin monostearate | 5.0 parts by weight |
| Eicosanyl behenoate | 1.0 part by weight |
| Liquid paraffin | 1.9 parts by weight |
| Trimethyrolpropane trioctanoate | 10.0 parts by weight |

The following ingredients except for ARRJ were admixed with the above mixture according to the following proportion and cooled to 30°C or lower. Further, ARRJ was admixed with the resultant according to the following proportion and emulsified using a homogenizer to produce skin cream for external use.

| | |
|---|---|
| 1,3-Butyleneglycol | 5.0 parts by weight |
| Aqueous solution of sodium lactate | 10.0 parts by weight |
| Methyl paraoxybenzoate | 0.1 part by weight |
| Peach-leaf extract | 1.5 parts by weight |
| Purified water | 62.2 parts by weight |
| ARRJ obtained by the method of Example A-3 | 1.0 part by weight |

Since the cream has a satisfactory moisture-retaining ability and allergic activity of royal jelly was reduced, there is no fear of causing allergic symptoms. Therefore, the cream is useful as a fundamental cosmetic for keeping freshness of skin.

## INDUSTRIAL APPLICABILITY

As is evident from the above, the present invention is based on an original knowledge that ARRJ whose water-soluble protein content in total proteins is reduced to less than 50% shows a remarkably low allergic activity and keeps the useful pharmacological action of royal jelly. Since ARRJ has no fear of causing significant allergic symptoms, it can be used by mammals including human easily and comfortably for keeping and increasing their health. Also, ARRJ of the present invention, having the above merits, can be advantageously used as various compositions such as foods, beverages, feeds, baits, pet foods, and cosmetics by incorporating with other ingredients.
The present invention has a remarkable effectiveness described above and is a significantly important invention that greatly contributes to this art.

**Claims**

1.   An allergen-reduced royal jelly, where the amount of water-soluble proteins is reduced to less than 50% (w/w) to the amount of total proteins.

2.   The allergen-reduced royal jelly of claim 1, where allergens are eliminated without substantially losing the useful pharmacological action of royal jelly.

3.   The allergen-reduced royal jelly of claim 1 or 2, where the weight ratio of the total proteins to 10-hydroxy-2-decenoic acid, on a free fatty acid basis, is less than six.

4.   A process for producing an allergen-reduced royal jelly comprising the steps of
            separating a royal jelly into a precipitate and a supernatant comprising water-soluble proteins by adding water and centrifuging;
            collecting low molecular substances by eliminating high molecular substances comprising water-soluble proteins from the resulting supernatant using ultra-filtration or gel filtration chromatography; and
            mixing the resulting low molecular substances with the aforesaid precipitate.

5. The allergen-reduced royal jelly of any one of claims 1 to 3, which is obtainable by the process of claim 4.

6. A composition comprising the allergen-reduced royal jelly of any one of claims 1 to 3 or claim 5.

7. A composition produced by incorporating the allergen-reduced royal jelly of any one of claims 1 to 3 or claim 5 into an anhydrous saccharide.

8. The composition of claim 7, wherein said anhydrous saccharide is any one of anhydrous trehalose, anhydrous maltose, and anhydrous cyclic tetrasaccharide.

9. The composition of any one of claims 6 to 8, further comprises saccharide transferred-vitamin C.

10. The composition of any one of claims 6 to 9, further comprises one or more antioxidant substances.

11. The composition of claim 10, wherein said antioxidant substances are flavonoids, polyphenols, vitamin E, and vitamin C.

12. The composition of any one of claims 6 to 11, which is in the form of a food, feed, bait, pet food, or cosmetic.

## INTERNATIONAL SEARCH REPORT

| International application No. |
| --- |
| PCT/JP03/11311 |

**A. CLASSIFICATION OF SUBJECT MATTER**
    Int.Cl$^7$  A23L1/076, A61K7/00, 7/48, A23K1/16

According to International Patent Classification (IPC) or to both national classification and IPC

**B. FIELDS SEARCHED**

Minimum documentation searched (classification system followed by classification symbols)
    Int.Cl$^7$  A23L1/076, A61K7/00, 7/48, A23K1/16

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 4-311357 A  (Api Kabushiki Kaisha),<br>04 November, 1992 (04.11.92),<br>Full text; particularly, Claim 2; examples 1 to 6<br>(Family: none) | 1-3,5,6,9-12<br>4,7,8 |
| X<br>Y | JP 2001-61418 A  (Pola Chemical Industries Inc.),<br>13 March, 2001 (13.03.01),<br>Full text; particularly, Claims 1, 4; Par. Nos.<br>[0008], [0009], [0011]<br>(Family: none) | 1-3,5,6,9-12<br>4,7,8 |
| Y | JP 2000-60455 A  (Api Kabushiki Kaisha),<br>29 February, 2000 (29.02.00),<br>Par. No. [0011]<br>(Family: none) | 4 |

[×] Further documents are listed in the continuation of Box C.      [ ] See patent family annex.

| * Special categories of cited documents: | "T" later document published after the international filing date or |
| --- | --- |
| "A" document defining the general state of the art which is not considered to be of particular relevance | priority date and not in conflict with the application but cited to understand the principle or theory underlying the invention |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 01 December, 2003 (01.12.03) | 16 December, 2003 (16.12.03) |

| Name and mailing address of the ISA/<br>    Japanese Patent Office | Authorized officer |
| --- | --- |
| Facsimile No. | Telephone No. |

Form PCT/ISA/210 (second sheet) (July 1998)

**EP 1 547 472 A1**

<table>
<tr><td colspan="2">INTERNATIONAL SEARCH REPORT</td><td>International application No.<br>PCT/JP03/11311</td></tr>
</table>

C (Continuation). DOCUMENTS CONSIDERED TO BE RELEVANT

| Category* | Citation of document, with indication, where appropriate, of the relevant passages | Relevant to claim No. |
|---|---|---|
| Y | US 5543513 A (Kabushiki Kaisha Hayashibara;<br>Seibutsu Kagaku Kenkyujo),<br>06 August, 1996 (06.08.96),<br>Full text; particularly, column 6, line 58 to<br>column 7, line 15<br>& EP 600730 A1          & AU 9352033 A<br>& CA 2110331 A          & CN 1094054 A<br>& TW 308555 A          & US 5693788 A<br>& JP 6-170221 A | 7,8 |
| A | JP 2002-112715 A (Api Kabushiki Kaisha),<br>16 April, 2002 (16.04.02),<br>Par. No. [0012]<br>(Family: none) | 1-12 |
| A | JP 11-146759 A (Kirin Beverage Kabushiki Kaisha),<br>02 June, 1999 (02.06.99),<br>Full text<br>(Family: none) | 1-12 |

Form PCT/ISA/210 (continuation of second sheet) (July 1998)